# EUROPEAN PATENT SPECIFICATION

(11) **EP 1 229 858 B1**
(45) Date of publication and mention of the grant of the patent: **23.05.2007**
(21) Application number: 00991907.7
(22) Date of filing: 30.10.2000
(51) Int. Cl.: A61L 27/58, A61L 27/44, A61L 27/46

(54) **BIODEGRADABLE POLYMER/CERAMIC IMPLANT MATERIAL WITH BIMODAL DEGRADATION PROFILE**
BIOLOGISCH ABBAUBARES POLYMER-/KERAMIK-IMPLANTATMATERIAL MIT BIMODALEMZERSETZUNGSPROFIL
MATERIAU D'IMPLANT BIODEGRADABLE EN POLYMERE/CERAMIQUE PRESENTANT UN PROFIL DE DEGRADATION BIMODAL

(30) Priority: 01.11.1999 US 162668 P
(43) Date of publication of application: 14.08.2002
(73) Proprietor: OSTEOBIOLOGICS, INC., San Antonio, Texas 78249 (US)
(72) Inventor: NIEDERAUER, Gabriele, G., San Antonio, TX 78259 (US); LEATHERBURY, Neil, C., San Antonio, TX 78258 (US); SLIVKA, Michael, A., Taunton, MA 02780 (US); KIESWETTER, Kristine, San Antonio, TX 78204 (US)
(74) Representative: Denness, James Edward
(86) International application number: PCT/US2000/041711
(87) International publication number: WO 2001/032072

(56) References cited:
- WO-A-98/46164
- US-A- 5 552 454
- US-A- 5 607 474
- US-A- 5 656 450
- US-A- 5 679 723
- US-A- 5 716 413
- US-A- 5 741 329

## Description

### CROSS-REFERENCE TO RELATED APPLICATIONS

This application claims priority to provisional application 60/162,668, filed November 1, 1999.

### BACKGROUND

In the United States, approximately 5-10% of the six million annual reported bone fractures progress to delayed unions or non-unions and require multiple procedures to help establish union of the fracture site. To aid the bone healing, defects can be treated with autograft bone, allograft bone, or synthetic bone graft substitutes. Of these procedures, only 10% are currently being addressed by synthetic grafting materials. Initially, patients with delayed unions or non-unions are treated by harvesting autologous bony tissue. The significant additional operative procedures needed to harvest autograft (Cornell, C.N. et al., "Multicenter trial of Collagraft as bone graft substitute," (1991) *J. Orthop. Trauma* 5:1-8) can raise the cost for each procedure by several thousand dollars, resulting in nearly 800 million dollars additional procedure costs. In comparison, market analysis has estimated the average cost of grafting material to be $825 per procedure.

Furthermore, the autologous bone harvest procedures result in considerable and often long-lasting pain, discomfort and numbness. Studies have shown that a patient from whom autologous bony tissue is harvested has an 8.6% chance of major complications and a 10.6% chance of minor complications (Younger, E.M. and Chapman, M.W., "Morbidity at bone graft donor sites," (1989) *J. Orthop. Trauma* 3(3):192-195). Donor site morbidity, which has been reported to be as high as 25%, is generally associated with risk of infection, significantly increased blood loss, significant postoperative pain and increased anesthesia time (Lane, J.M. and Bostrom, M.P.G., "Bone grafting and new composite biosynthetic graft materials," (1998) In American Academy of Orthopaedic Surgeons Instructional Course Lectures, W.D. Cannon, Jr., editor, pp. 525-534). Allograft bone is derived from cadavers and carries the potential of infectious agent transfer and varies significantly in bone induction ability (Schwartz, Z. et al., "Ability of commercial demineralized freeze-dried bone allograft to induce new bone formation," (1996) *J. Periodontol.* **67**:918-926).

Bone healing is a sequential process that involves several steps (see Figure 3). As damage occurs at the site, through fractures or removal of bone due to surgical excision as in the case of lesions, bone necrosis occurs in the adjacent bone tissue due to changes in blood and nutrient supply (Ham, A.W. and Cormack, D.H., "Bone and bones," (1979) In Histophysiology of Cartilage, Bone and Joints, Anonymous, pp. 450-456). After the injury, a hematoma forms, and the mitogenic activity associated with new bone formation increases over the first four weeks leading to a bridging woven callus which forms at about six weeks. During this time frame, considerable upregulation of molecules involved in cartilage formation and endochondral ossification occurs and immature lattice woven bone forms due to the wound healing response. Although the fragments of the fracture are joined together, it is of relatively low strength. Substantial union of the site is initiated at this time but does not significantly occur until lamellar bone starts filling in this lattice structure to form a compact load-bearing structure. This progress has been shown to occur at about 18 weeks in humans (Roberts, W.E., "Bone tissue interface," (1988) *J. Dent. Ed.* **52**:804-8-9) and may take up to a year to complete (Ham and Cormack, 1979, *supra).*

The commercially available first generation of synthetic bone substitute devices consists of materials that resorb over time frames that are not in sync with the time frame of normal bone repair. The calcium phosphate ceramics and cements and their collagen-containing composites can remain in situ for up to several years. This is much longer than needed. Calcium sulfate materials, on the other hand, resorb within two months and are not available for the bulk of the fracture healing process. Finally, the majority of the currently-available products are indicated for filling of bony defects. In short, they serve as osteoconductive scaffolds for bone repair but are not recommended for any type of load bearing.

In the United States alone there are over 450,000 bone graft procedures annually. Spinal and general orthopaedic fractures account for over 85% of all grafting procedures. The ability to provide surgeons with a material that they can utilize in the role of autograft on more difficult fractures would result in a considerable health care cost savings due to decreased surgical time, decreased requirements for replacement blood and a considerable benefit to patients from decreased morbidity. Further, if the implant supported partial to full weight bearing, the patient would be able to ambulate more quickly and increase the possibility of returning to work earlier. The use of a synthetic bone substitute material which can fully degrade, which promotes bone formation by supplying a source of bone-friendly ions, and which can support limited weight bearing would have considerable clinical appeal. Furthermore, a material that can be shaped and formed in the surgical suite will allow the clinician to customize it for each specific case.

Cartilage repair is also a challenging clinical problem because once adult cartilage sustains damage, be it traumatic or pathologic, an irreversible, degenerative process can occur (Newman, A.P., "Current concepts: Articular cartilage repair," [1998] *Am. J. Sports. Med.* **26**:309-324). The resulting defects may further lead to osteoarthritis (Newman, 1998, *supra;* Buckwalter, J.A. and Mankin, H.J., "Articular cartilage: Degeneration and osteoarthritis, repair, regeneration and transplantation, [1998] In American Academy of Orthopaedic Surgery Instructional Course Lectures, W.D. Cannon, Jr., ed., Rosemont, Am. Academy of Orth. Surgeons, pp. 487-504). Attempts to repair articular cartilage have included implantation of artificial matrices, growth factors, perichondrium, periosteum and transplanted cells, but to date no reliable, reproducible approach has been identified.

Repair of osteochondral defects involves two types of distinct tissues, articular cartilage and subchondral bone. In designing a multiphase implant, the healing of the underlying subchondral area of the defect site is critical to support the overlying neocartilage regenerate. Over the last decades, the use of bioactive glasses, calcium phosphates and similar ceramics for bone repair has shown their ability to bond to bone and accelerate bone healing (Hulbert, S. et al., "Ceramics in clinical applications, past, present, and future," [1987] In: High tech ceramics, P. Vinvenzini, ed., Amsterdam, Elsevier Science publishers, pp. 3-27; Hench, L.L., "Bioactive Implants," [1995] *Chemistry and Industry* **14**:547-550; Jarcho, M., "Biomaterial aspects of calcium phosphates: Properties and applications," [1986] *Dental clinics of North America* **30**(1):25-47; deGroot, K. et al., "Significance of the porosity and physical chemistry of calcium phosphate ceramics," [1988] *Ann. N.Y. Acad. Sci.* **523**:272-277). However, for subchondral bone repair in rabbit and goat osteochondral defects, bioactive glass and hydroxyapatite have led to mixed results.

Suominen et al. ("Subchondral bone and cartilage repair with bioactive glasses, hydroxyapatite, and hydroxyapatite-glass composite," [1996] *J. Biomater. Mater. Res.* **32**:543-551) treated 4 x 4 mm osteochondral defects in rabbit femurs with bioactive glass, hydroxyapatite and hydroxyapatite-glass and reported the formation of lamellar subchondral bone with restoration of hyaline-like cartilage surface after 12 weeks. On the other hand, van Susante et al. (1998) "Chondrocyte-seeded hydroxyapatite for repair of large articular cartilage defects. A pilot study in the goat," *Biomaterials* **19**:2367-2374, attempted to restore 10 mm cartilage defects in goat femurs with chondrocytes suspended in fibrin glue on top of hydroxyapatite cylinders. Due to inadequate fixation of the implant, fibrocartilaginous repair tissue resulted.

Biodegradable polymers, specifically polylactide-co-glycolides, are completely synthetic, resorb naturally within months, and have a long history of safe and effective use in other medical applications. Because these polymers degrade into products which are naturally found in the body and are eliminated through normal physiologic pathways, many studies have shown that polylactic acid (PLA), polyglycolic acid (PGA), and poly-lactic-polygalactic acid (PLG) polymers are biocompatible and non-toxic materials (Kumta, S.M. et al., "Absorbable intramedullary implants for hand fractures, animal experiments and clinical trials," [1992] *J. Bone Joint Surg.* **74-B**:563-566; Bucholz, R.W. et al., "Fixation with bioabsorbable screws for the treatment of fractures of the ankle," [1994] *J. Bone Joint Surg.* **76-A**:319-324).

Biodegradable polymers were first introduced in the 1970s as biodegradable sutures such as Vicryl^{®} and Dexon^{®}. Since that time, these materials have been utilized in commercially available devices that are used for applications ranging from interference screws to tacks and from pins for ligament and tendon healing to fracture fixation of low-load bearing craniofacial fractures. One of the key advantages of this family of materials is that the degradation rate can be tailored to range from approximately two weeks to several years. The mechanical properties can also be tailored as a function of the polymer's molecular weight, processing, composition and crystallinity (Engelberg, 1. and Kohn, J., "Physio-mechanical properties of degradable polymers used in medical applications: A comparative study," [1991] *Biomaterials* **12**:292-304; Eling, B. et al., "Biodegradable materials of poly(L-lactic acid): 1. Melt-spun and solution spun fibers," [1982] *Polymer* **23**:1587-1593).

Bioactive ceramics are another class of materials that have been shown to be highly biocompatible. These materials are well characterized and can be surface active and/or resorbable (deGroot, K., Biocompatibility of clinical implant materials, [1981] D.F. Williams, editor, pp. 199-222). They are generally comprised of inorganic ions that are similar to actual components of the inorganic matrix of bone and can provide an ample source of ionic components familiar to bone cells (deGroot, 1981, *supra).* Several studies have shown that these materials are capable of bonding directly to soft and hard tissue and accelerating bony healing (Hulbert, S. et al., "Ceramics in clinical applications, past, present, and future," [1987] In High tech ceramics, P. Vinvenzini, editor, pp. 3-27; Hench, L.L., "Bioactive implants," [1995] *Chemistry and Industry* **14**:547-550; Jarcho, M., "Biomaterial aspects of calcium phosphates: properties and applications. Reconstructive implant surgery and implant prosthodontics," [1986] *Dent. Clin. North Am.* **30**:25-47; deGroot, K. et al., "Significance of the porosity and physical chemistry of calcium phosphate ceramics," [1988] *Ann. New York Acad. Sci.* **523**:272-277). The use of bioceramics in orthopaedic applications has been reported since the 1890s (Dreesman, H., "Ueber Knochenplombierung," [1892], *Bier Klin. Chir.* **9**:), but their use has been limited due to their inherently brittle nature.

Biodegradable implant materials known to the art include those disclosed and referenced in U.S. Patent Nos. 5,607,474, issued March 4, 1997, 5,397,572 issued March 14, 1995, 5,741,329 issued April 21, 1998, 5,876,452 issued March 2, 1999, 5,290,494 issued March 1, 1994, 5,656,450 issued August 12, 1997, 5,716,413 issued February 10, 1998, 5,863,297 issued January 26, 1999, 5,492,697 issued February 20, 1996, and PCT Publications WO 98/53768 published December 3, 1998, WO/98/24483 published June 11, 1998 and WO 98/46164 published October 22, 1998.

An implant material is needed which is biodegradable in a manner consistent with osteochondral healing and is capable of bearing weight and promoting rapid healing of bone and cartilage defects.

### SUMMARY

This invention provides a biodegradable implant material having a selected biphosic degradation profile comprising a biodegradable polymer having a molecular weight between 25,000 and 1,000,000 Daltons and having substantially uniformly distributed therein a biodegradable ceramic selected from the group consisting of calcium sulphate and calcium phosphate. Preferably the implant material is substantially nonporous (fully dense), which means the percent porosity is less than about five percent.

Methods of making substantially nonporous therapeutic implant materials are also provided comprising preparing said polymer in uncured form, mixing particles of a biodegradable ceramic into said polymer, and applying heat and pressure to said mixture to produce a substantially nonporous, cohesive implant material. Methods of making porous implants comprising dissolving out the biodegradable ceramic materials *in vivo* or *in vitro* are also provided, as are the porous implants themselves.

### BRIEF DESCRIPTION OF THE FIGURES

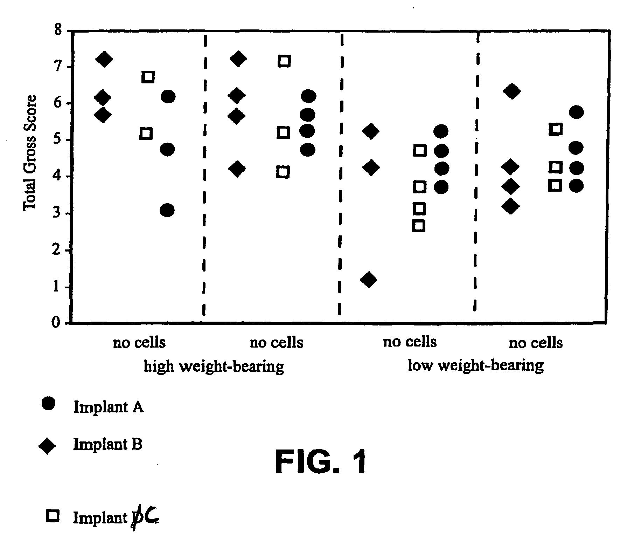

Figure 1 plots gross scores based on edge integration, cartilage surface, fill area, and color/opacity for four implant constructs as follows: ● Implant A: 75:25 polylactic/polyglycolic acid for the cartilage phase and 75:25 polylactic/polyglycolic acid for the bone phase; ◇Implant B: 75:25 polylactic/polyglycolic acid with 10% fiber reinforcement for the cartilage phase and 75:25 polylactic/polyglycolic acid with 20% fiber reinforcement for the bone phase; ▲ Implant C: 75:25 polylactic/polyglycolic acid with 10% fiber reinforcement for the cartilage phase and 55:45 polylactic/polyglycolic acid with 20% Bioglass^{®} for the cartilage phase; ■ Implant D: 75:25 polylactic/polyglycolic acid with 10% fiber reinforcement for the cartilage phase and 75:25 polylactic/polyglycolic acid with 50% medical grade calcium sulfate for the bone phase.

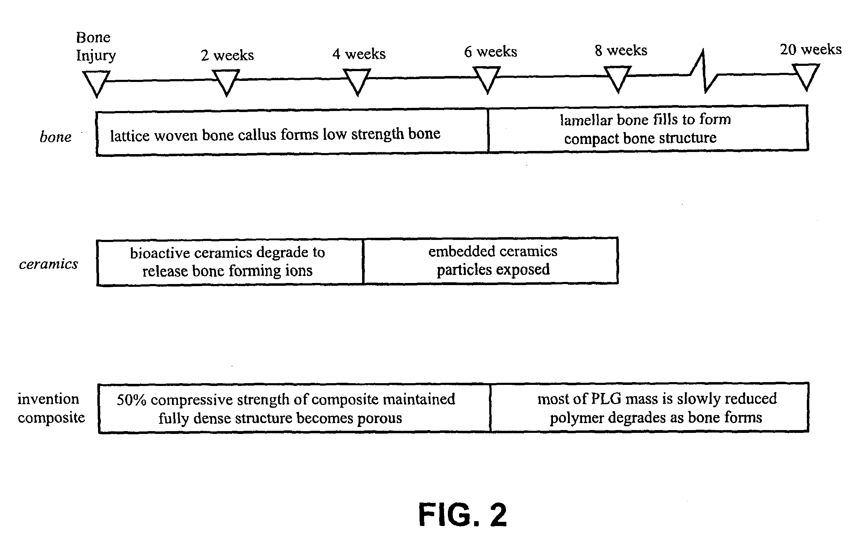

Figure 2 is a timeline in weeks showing expected bone healing in injured bone (1) without any treatment; (2) treated with prior art ceramic implants, and (3) treated with the implant material of this invention.

### DETAILED DESCRIPTION

The term "biodegradable" means capable of breaking down over time inside a patient's body or when used with cells to grow tissue outside the body. A therapeutic implant is a device used for placement in a tissue defect in a patient (human or animal) to encourage ingrowth of tissue and healing of the defect. Implants of this invention may comprise cells.

Polymers known to the art for producing biodegradable implant materials may be used in this invention. Examples of such polymers are polyglycolide (PGA), copolymers of glycolide such as glycolide/L-lactide copolymers (PGA/PLLA), glycolide/trimethylene carbonate copolymers (PGA/TMC); polylactides (PLA), stereocopolymers of PLA such as poly-L-lactide (PLLA), Poly-DL-lactide (PDLLA), L-lactide/DL-lactide copolymers; copolymers of PLA such as lactide/tetramethylglycolide copolymers, lactide/trimethylene carbonate copolymers, lactide/δ-valerolactone copolymers, lactide ε-caprolactone copolymers, polydepsipeptides, PLA/polyethylene oxide copolymers, unsymmetrically 3,6-substituted poly-1,4-dioxane-2,5-diones; poly-β-hydroxybutyrate (PHBA), PHBA/β-hydroxyvalerate copolymers (PHBA/HVA), poly-β-hydroxypropionate (PHPA), poly-p-dioxanone (PDS), poly-δ-valerolatone, poly-∈-caprolactone, methylmethacrylate-N-vinyl pyrrolidone copolymers, polyesteramides, polyesters of oxalic acid, polydihydropyrans, polyalkyl-2-cyanoacrylates, polyurethanes (PU), polyvinyl alcohol (PVA), polypeptides, poly-β-maleic acid (PMLA), and poly-β-alkanoic acids.

Preferred biodegradable polymers for use in making the materials of this invention are known to the art, including aliphatic polyesters, preferably polymers of polylactic acid (PLA), polyglycolic acid (PGA) and mixtures and copolymers thereof, more preferably 50:50 to 85:15 copolymers of D,L-PLA/PGA, most preferably 75:25 D,L-PLA/PGA copolymers. Single enantiomers of PLA may also be used, preferably L-PLA, either alone or in combination with PGA.

The biodegradable polymer in the implant material of this invention has a molecular weight between 25,000 and 1,000,000 Daltons, preferably between about 40,000 and about 400,000 Daltons, and most preferably between about 55,000 and about 200,000 Daltons.

The biodegradable ceramics of this invention may include highly purified, preferably medical grade, calcium sulfate, used in particulate form having a particle size between about 100 and about 1000 µm, and more preferably between about 250 and about 850 µm. Other suitable biodegradable ceramics include calcium phosphate.

Methods of making the implant materials of this invention are provided comprising mixing particles of the biodegradable polymer with particles of the biodegradable ceramic and applying heat and pressure to the mixture to produce a substantially uniform cohesive implant material. The term "uniform" means that substantially any randomly-selected portion of the volume of the material (large enough to contain multiple particles of the biodegradable ceramic) will have the same composition and properties as any other portion. Such uniform materials have the biodegradable particles "uniformly distributed" therein. The term "cohesive" means that the implant material is nonfriable and will not fracture under conditions of ordinary use, including implantation into locations requiring weight bearing. Preferably the process is conducted at a temperature sufficient to at least partially melt the biodegradable polymer. For the polylactic:polyglycolic (PLA:PGA) polymer composition of the preferred embodiments, preferably the temperature is between about 75°C and 100°C. The pressure is sufficient to compact the material and eliminate air, and preferably is between about 10 and about 100 ksi and more preferably is between about 20 and about 50 ksi. The heat and pressure may be applied in a heated mold such as a hydraulic press such as that of Carver, Inc., Wabash, IN. They may also be applied by means of an extrusion molding device such as a single-screw melt extruder such as that of Randcastle Company, Cedar Grove, N.J.

The particles of biodegradable polymer should be fine enough to assure homogenous dispersion with the ceramic, and have an average size preferably below about 60 mesh.

The particles of biodegradable ceramic have an average size preferably below about 10-300 mesh, more preferably between about 20 and about 60 mesh. Preferably these particles are spheroid. They may also be irregularly shaped, such as fibers or ellipsoids.

The compositions of this invention preferably have a volume ratio of biodegradable ceramic to biodegradable polymer of between about 10:90 and about 70:30, preferably ceramic is present at about 20-50 volume percent.

Porous implant materials of this invention may be made by curing the mixture of biodegradable polymer and particles of biodegradable ceramic under conditions of heat, pressure and vacuum sufficient to form pores, or by exposing the non-porous materials to *in vivo* or *in vitro* conditions causing dissolution of the biodegradable ceramic more rapidly than the biodegradable polymer, thus leaving void spaces for ingrowth of cells.

Such porous implant materials are also provided herein. The porosity is tailored by selection of the polymer to ceramic ratios and their particle sizes.

The implant materials of this invention preferably have mechanical properties similar to the inherent mechanical properties of the recipient tissue, e.g., the Young's modulus is preferably between about 1 GPa and about 30 GPa for applications to cancellous bone and partial weight-bearing areas of bone; the Young's modulus is preferably between about 5 GPa and about 30 GPa for applications to compact bone and full weight-bearing areas of bone.

Cylinders, wafers, spheres, strips, films, and irregularly-shaped implants, as well as particulate bone-graft materials containing biodegradable ceramics are provided herein, as are biodegradable polymeric hand-shapable materials containing biodegradable ceramics and biodegradable polymeric materials capable of continuous, smooth release ofbioactive agents and containing biodegradable ceramics.

The implant materials of this invention may be used by forming said materials into implant devices selected from the group consisting of: tissue scaffolds with and without cells, granular bone graft substitute material, multi-phase osteochondral implants, weight-bearing bone implants, no- to low-weight-bearing implants or fixation devices, tacks, pins, screws, bone onlays, and films. Multi-phase implants are described, e.g., in U.S. Patent No. 5,607,474 issued March 4, 1997.

The implant materials of this invention have a bimodal degradation profile, which means the biodegradable ceramic degrades first. The biodegradable ceramic degrades about twice as fast as the polymer. The exact degradation times are governed by the selection of the polymer and ceramic and by the metabolism of the surrounding tissue. Thereby, the biomodal degradation profile can be tailored for the intended application. For example, for subchondral or cancellous bone repair, the biodegradable ceramic substantially degrades within about four to about eight weeks and the biodegradable polymer substantially degrades within about sixteen to about twenty weeks.

The implant materials of this invention may also include fibers, preferably biodegradable fibers as described in PCT Publication WO 98/53768 published December 3, 1998. Fibers are preferably present in an amount between about 0 and about 60, more preferably between about 10 and about 50 volume percent.

Fibers are examples of reinforcing components useful in the implant materials of this invention. The reinforcing component is suspended in a continuous matrix and can be either fibrous or particulate in nature. Fiber reinforcement can be used to give anisotropic, or directional stability to materials, in particular imparting good bending and tensile properties. Particulate reinforcement generally produces isotropic, or non-directional strength which performs particularly well in compression.

The implant material of this invention may also be hand shapable at body temperatures in accordance with U.S. Patent No. 5,863,297.

The implant materials of this invention are suitable for implantation into bone, into cartilage or both bone and cartilage, e.g. with mechanical properties matching both bone and cartilage as taught in U.S. Patent No. 5,607,474.

Implants made from these materials may be covered with films as described in PCT Publication WO/98/24483 published on June 11, 1998 or may be formed into films and other devices in accordance with the disclosure thereof and PCT Publication No. WO 98/46164 published October 22, 1998.

Calcium sulfate increases the storage modulus and slow the degradation of poly-D,L-lactide-co-glycolide (DL-PLG), and reduces the degradation pH, leading to a more acidic degradation profile. Materials composed of 60% calcium sulfate powder (< 150 µm) and 40% 75/25 DL-PLG retain 31 % of their initial storage modulus over 5 weeks degradation at 37° C, going from 3.46 GPa to 1.08 GPa. Materials fabricated of large particle size (250-850 µm and 850-2000µm) calcium sulfate (OsteoSet^{™} Pellets) exhibit a lower initial storage modulus relative to a pure PLG control. At five weeks degradation, they retain a greater percentage of the initial value. In all cases, the softening point (or physical transition point) of the materials is at or below 37° C. OsteoSet^{™} Pellets tend to reduce the pH of the degradation solution more rapidly than pure calcium sulfate powder.

### EXAMPLES

### Implant Manufacture and Characterization

Multiphase implant prototypes were prepared using poly(D,L-lactide-co-glycolide) (75:25 PLG or 55:45 PLG) as the base material. PGA fibers (FR), and medical grade calcium sulfate (MGCS) were used as additives to vary stiffness and chemical properties (Table 1). The sterilized implants consisted of a bone phase (1.2 mm), a cartilage phase (2.7 mm), and a thin solid film (0.1 mm) on top. Thickness of the cartilage phase was determined by the average cartilage thickness at identical sites from non-study animals. ,

**Table 1. Implant constructs, including type of phase and stiffness (in GPa), tested under physiological conditions. All constructs included a thin film layer above the cartilage phase.**

| **Implant** | **Cartilage Phase** | **Stiffness** | **Bone Phase** | **Stiffness** |
|---|---|---|---|---|
| **A** | 75:25 PLG | 12±1.5 | 75:25 PLG | 12±1.5 |
| **B** | 10% FR*-75:25 PLG | 32±2.1 | 20% FR-75:25 PLG | 48±5.4 |
| **C** | 10% FR-75:25 PLG | 32±2.1 | 75:25 PLG+ 50%MGCS*** | 1080±484 |

| | | | | |
|---|---|---|---|---|
| * Fiber-Reinforced *** Medical Grade Calcium Sulfate (fully dense - all others were 60-70% porous). | | | | |

The constructs consisted of three layers: a thin, fully dense film on the articulating surface, a porous cartilage phase, and porous or fully dense bone phase, depending on implant type. Each phase was prepared separately as detailed below. When bone and cartilage phases of the implants were identical, they were prepared as a single phase.

### 75:25 PLG preparation

A resorbable polylactic/polyglycolic acid (PLG) copolymer with a ratio of 75 D,L-PLA:25 PGA (Boehringer Ingelheim, Ingelheim, Germany) was dissolved in acetone and then precipitated with ethanol. The precipitated gel mass was kneaded and expanded under vacuum and elevated temperature to produce a porous construct.

### FR-75:25 PLG preparation

To prepare the fiber-reinforced composites, 75 D,L-PLA:25 PGA polymer was dissolved in acetone. Polyglycolic acid fibers (FR) (Albany International, Mansfield, MA) at a concentration of 10 or 20 weight percent were dispersed in ethanol and mixed with the dissolved polymer to precipitate the matrix. The precipitated gel was kneaded to disperse and preferentially orient the fibers and expanded under vacuum and elevated temperature, resulting in a porous scaffold.

### PLG Film preparation

Thin films composed of 75 D,L-PLA:25 PGA were prepared with a thickness range of 100 ± 30 µm. Finely ground polymer was placed between two sheets of aluminum foil and pressed on preheated platens of a laboratory press at 250 ± 30°F to produce a fully dense layer.

### Assembly of implants

To assemble the various phases of the implants, porous stock materials were cut to a thickness of either 1.2 mm intended for the cartilage phase, 2.7 mm intended for the bone phase, or 3.9 mm for single phases. Using a small amount of solvent, the various phases were "glued" together. A coring tool was used to punch out the implants to a diameter of 3.0 mm. After manufacture, gas chromatography was performed on each material construct to confirm that residual solvent levels were less than 100 ppm. Assembled implants were sterilized using ethylene oxide and aerated until residual sterilant levels were below acceptable levels.

### Characterization

Gel permeation chromatography was used to measure the weight average molecular weight (M_{w}) and polydispersity of the two PLG materials utilized. Because the PGA fibers do not dissolve in the chromatography solvent, their molecular weight was not assessed. Porosity of the prepared materials was calculated from the volume and mass of sample specimen. Compressive stiffness of the implant materials was determined using unconfined, parallel plate compression at 0.1 mm/mm/min under physiological conditions.

### Implantation

Custom made tooling was utilized to create precise osteochondral defects 3 mm in diameter and 4 mm in depth in 16 Spanish goats (22-38 kg). To vary the load bearing environments, defects were located in the medial femoral condyle and the medial border of the patellar groove. Half of the implants were loaded with autologous costochondral chondrocytes (30,490±11,000 cells) isolated 48 hr prior to surgery from the cartilaginous portion of the 11^{th} and 12^{th} ribs. Bilateral defect sites were randomly treated and allowed to heal for 16 weeks. All animals were fully weight bearing immediately after surgery. At euthanasia, gross scoring criteria (edge integration, surface roughness, defect fill and color/opacity, max score=8). Decalcified histological sections were taken at approximately the edge, part, and center of the defect and stained with H&E and SafraninO/Fast Green. Sections were also processed through hyaluronidase digestion to analyze collagen architecture. Sections were blindly evaluated by an independent pathologist using a scale adapted from Frenkel *et al.* and Caplan *et al.,* The scale (max score = 25) characterized the nature of the predominant tissue (0-4), the structural characteristics (surface 0-3, homogeneity 0-2, thickness 0-2, bonding 0-2), cellular changes of degeneration in the defect area (0-4) and adjacent cartilage (0-3), and subchondral bone reconstruction (0-5). Statistics were performed using nonparametric analyses.

To grade the nature and organization of the repair tissue in the cartilage region in more detail, metachromatic staining and collagen architecture evaluations were conducted and their correlation, if any, determined.

**Table 2. Grading scale for Safranin-O staining and Polarized Light analysis of the repair cartilage.**

| **Score** | **Safranin-O Staining (SO)** | **Polarized Light Analysis (PL)** |
|---|---|---|
| 4 | Normal | Hyaline/Normal |
| 3 | Near Normal | Hyaline/Disorganized |
| 2 | Moderate/Mixed | Mixed Fibrillar/Hyaline |
| 1 | Slight/Scarce | Fibrillar/Organized |
| 0 | None | Fibrillar/Disorganized |

Using the scoring scale described in Table 2, five blinded observers scored randomly ordered slides for Safranin-O staining and polarized light analysis. Normal articular cartilage sections were used as reference positive controls. Linear regression was used to correlate the Safranin-O scores with the scores from polarized light analysis. The scores from all five observers for each sample were added for a maximum total score of 20, for each the Safranin-O and polarized light analysis.

### Results

For all results related to the *in vivo* portion of the study, no statistically significant differences were found among the animals or left/right side.

### Implant Characterization:

Relative to polystyrene standards, the weight average molecular weight (M_{w}) and polydispersity (Pd) was 70 kDa and 1.7 for the 55:45 PLG, and 90 kDa and 1.8 for the 75:25 PLG. Porosity for all of the porous constructs ranged between 60 and 70%. Results from the mechanical testing for cartilage and bone phases are given in Table 1. For the cartilage phase materials, reinforcing the porous PLG scaffolds with 10 % PGA fibers (Implants B, and C) significantly increased the compressive modulus over the neat scaffold (Implant A). For the bone phase materials, the compressive modulus for each formulation was significantly different from each other. The bone phase of implant type C was by far the stiffest and closest to the range of properties previously reported for cortical and trabecular bone.

### Gross Observations:

Animals tolerated the bilateral surgeries well, and all animals were ambulatory immediately following recovery from the anesthesia. The gross necropsies of the major organs and lymph nodes indicated no abnormalities related to the implants. The gross examinations of the knee joint showed that there were no abrasions on the opposing articulating surfaces, and no inflammation of the synovial membrane and other joint tissues was noted. Visual observations generally showed that new tissue integrated well with the native cartilage, that the surface of the repair site was fairly smooth, and that the defects were almost entirely filled with repair tissue of similar color and texture to the adjacent normal cartilage. Thin, elongated fissures in the neocartilage were more prominent in the condyle than the patellar groove. Repair tissue in the patellar groove was more opaque and exhibited less complete resurfacing than in the condyle.

The sum of the three individual categories (gross scores, edge integration, cartilage surface, fill area, and color/opacity) are plotted in Figure 1. Gross scoring indicated no statistically significant differences between addition/omission of cells or between implant types. Gross scoring did show a significantly (p<0.0001) higher total score for defect healing on the condyle (5.7±0.98) than in the patellar groove (4.1±0.96). Differences between condyle and patellar groove were primarily found for the edge integration and cartilage surface categories. The mean gross scores for the various implant types ranked as follows: in the condyle, implant types B=6.0, C=5.8, A=5.3; in the patellar groove, implant types A=4.3, C=3.9, B=3.8.

### Histology Results; H&E Grading:

Overall qualitative evaluations of the histology slides showed that all groups had a high percentage of hyaline cartilage and good bony restoration. Reproducibility of repair tissue histology was quite consistent within a given treatment group, and in most cases a group of four animals was sufficient to determine the overall effects of a given treatment on osteochondral defect repair. Integration of healed tissue showed excellent bonding with the native cartilage, at times making it difficult to identify the original defect margins. The repair cartilage thickness was very close to that of adjacent cartilage. Overall, little to no cartilage surface fibrillation was noted. Most sections showed no residual implant material, and if present, it was located near the bottom of the original defect perimeter in the lower portion of the subchondral bone. Inflammation was always subchondral and associated at some level with all of the implants. Healing varied throughout the defect location (edge, part, center), with the best healing occurring at the edge of the defect and the worst healing occurring at the center of the defect. Fissures were noted more frequently in the repair tissues of the condyle than in the patellar groove. Fissures were mostly, but not always, observed near the center of the cartilage repair tissue. The presence of a small zone of acellular tissue and fibrocartilage adjacent to the fissures suggests that this is a focal structural defect rather than a failure to bond with adjacent tissues. Implant A exhibited more chondrocyte clustering and had the lowest content of hyaline cartilage overall. Implant B as more likely to exhibit hypocellular or hypercellular repair tissue, with degenerative changes occurring more frequently in the adjacent articular cartilage. Implant B also showed the poorest subchondral bone reconstruction. Implant C showed the most complete repair tissue bonding and most normal repair tissue cellularity.

**Table 3. Mean and Standard Deviation of the Histology Grading Scores**

| Treatment | Implant A | Implant B | Implant C |
|---|---|---|---|
| High WB*/No Cells | 16.0±2.4 | 15.8±1.9 | 15.3±2.2 |
| High WB/Cells | 13.8±2.4 | 15.0±1.2 | 16.8±2.6 |
| Low WB/No Cells | 8.8±2.5 | 9.3±3.7 | 10.3±3.8 |
| Low WB/Cells | 14.3±7.46 | 10.8±4.7 | 12.8±3.8 |

| | | | |
|---|---|---|---|
| * Weight-bearing | | | |

Total histology grading scores, presented in Table 3, were not significantly different for the addition/omission of cells or for the different implant types. The total scores were significantly better (p<0.0001) for the healing of defects in the condyle than for the patellar groove. In both locations, the ranking based on mean total scores for the implant types were as follows: C > B > A.

The external pathology review of the histology concurred with the internal review for all treatment factors, except the high/low weight bearing. For histology scoring, the external evaluation showed no significant differences for defect healing between the condyle and patellar groove. However, for all other treatment factors, implant type and addition/omission of cells, results from the internal and external evaluations concurred. With respect to implant type, the pathologist also ranked implant type C as having the best cartilage tissue repair.

### Safranin-O and Collagen Architecture:

A direct correlation was found between the Safranin-O (SO) scores and the Polarized Light (PL) scores (R=0.81) with mean SO and PL scores presented in Table 4.

**Table 4. Mean and Standard Deviation of Safranin-O and Polarized Light Scores**

| **Safranin-O Scores** | | | |
|---|---|---|---|
| Treatment | Implant A | Implant B | Implant C |
| High WB/No Cells | 17.5±3.3 | 19.5±1.0 | 18.0±1.8 |
| High WB/Cells | 17.0±1.6 | 18.5±1.3 | 19.0±0.8 |
| Low WB/No Cells | 10.5±3.9 | 8.5±4.4 | 7.3±3.9 |
| Low WB/Cells | 12.8±1.5 | 12.0±2.2 | 10.5±2.5 |

| **Polarized Light Scores** | | | |
|---|---|---|---|
| Treatment | Implant A | Impant B | Impant C |
| High WB/No Cells | 16.5±2.9 | 18.3±2.9 | 17.3±1.9 |
| High WB/Cells | 15.5±0.6 | 18.3±1.7 | 18.5±1.0 |
| Low WB/No Cells | 11.8±4.0 | 8.5±4.2 | 8.3±2.2 |
| Low WB/Cells | 8.3±3.3 | 8.3±3.2 | 10.3±3.8 |

Both scores revealed no significant differences between addition/omission of cells or among implant types. However, each score revealed significantly better (p<0.0001) healing in the condyle (means SO = 18.0, PL = 17.3) than the patellar groove (means SO = 10.6, PL = 8.8). Representative sections showed that uniform, intense staining with Safranin-O frequently corresponded to an organized, hyaline collagen architecture. These characteristics were predominant in the repair tissue found in the condyles. In contrast, weak Safranin-O staining frequently occurred where the collagen was fibrous or disorganized. However, some cases were noted where a sample had uniform Safranin-O staining and the collagen architecture appeared fibrous. Whereas the Safranin-O staining was variable in the repair tissue found in the patellar groove, the collagen architecture was mostly fibrous and/or disorganized. Total gross scores correlated well with Safranin-O staining R = 0.71) and polarized light scoring R = 0.78), showing that the total gross score is a good indicator of the nature of the repair tissue as assessed by proteoglycan content and collagen architecture.

### Discussion

The current investigation demonstrates that focal, osteochondral defects in the high weight-bearing and low weight-bearing regions of distal femurs treated with various implant constructs were repaired with hyaline-like cartilage and good underlying bone. Numerous publications have shown that untreated, osteochondral defects in large animals do not spontaneously regenerate with "perfect" tissue.

The high and low load-bearing regions of the distal femur of various species have been characterized both histologically and mechanically. For rabbits, monkeys and dogs, articular cartilage is thicker in the high weight-bearing areas than that in less-weight-bearing areas which concurs with what we observed in this study for the goat model. Low-weight bearing areas differ biochemically from high-weight bearing areas. Because results from the gross observations, the histology grading scale and the Safranin-O/Collagen architecture correlation all showed significantly better healing in the condyle than the patellar groove, it appears that the implant construct design of comparable compressive stiffness and preferential alignment of the scaffold architecture is more amenable to repair in the condyle than the patellar groove. This observation was further supported in the condyle by the higher ranking of the scaffolds with stiffer cartilage phases (Implants B and C,) than the control scaffold (Implant A). Thus a cartilage phase with stiffer mechanical properties (similar to high weight bearing cartilage) enhances articular cartilage healing.

As to the repair of the subchondral bone, even though the three implants differed in bone phase composition and stiffness, we found no significant differences in their overall healing scores. Implant D, which included calcium sulfate, ranked the highest in the total histologic grading score and in the qualitative observations. Consequently, the addition of a bioactive biodegradable ceramic in the bone phase of the constructs has a beneficial effect on overall osteochondral healing.

In this screening study, histological characterization of the articular cartilage repair sites treated with three multiphase implants showed that an implant constructs with a fiber-reinforced cartilage phase and a 75:25 PLG / MGCS resulted in a higher mean scores. Results of this study support our hypothesis that a cartilage phase with stiffer mechanical properties enhances articular cartilage healing. Furthermore, the combination of a bioactive ceramic, such as calcium sulfate, with a faster degrading polymer for the bone phase also appears to have a beneficial effect on healing.

## Claims

1. A biodegradable implant material having a selected biphasic degradation profile comprising a biodegradable polymer having a molecular weight between 25,000 and 1,000,000 Daltons and having substantially uniformly distributed therein a biodegradable ceramic selected from the group consisting of calcium sulfate and calcium phosphate.

2. The implant material of claim 1 which is substantially nonporous.

3. The implant material of claim 1 wherein said biodegradable polymer comprises a copolymer of polylactic acid and polyglycolic acid.

4. The implant material of claim 1 wherein said biodegradable ceramic degrades twice as fast as the biodegradable polymer.

5. The implant material of claim 1 wherein said biodegradable ceramic particle size is initially between 100 and 2000 µm.

6. The implant material of claim 2 having a mechanical property similar to the corresponding inherent mechanical property of the recipient tissue.

7. The implant material of claim 1 also comprising biodegradable fibers.

8. The implant material of claim 1 which is hand shapable at between body temperature and 55°C.

9. A method of making a biodegradable implant material of claim 1 comprising:
a) mixing particles of a selected biodegradable polymer having a molecular weight between 25,000 and 1,000,000 Daltons with particles of a selected biodegradable ceramic selected from the group consisting of calcium sulfate and calcium phosphate; and
b) applying heat and pressure to said mixture to produce a substantially uniform cohesive implant material.

10. The method of claim 9 wherein said temperature is between 75 and 100°C.

## Patentansprüche

1. Ein biologisch abbaubares Implantatmaterial mit einem ausgewählten zweiphasigen Zersetzungsprofil aufweisend ein biologisch abbaubares Polymer mit einem Molekulargewicht zwischen 25 000 und 1 000 000 Daltons und mit im Wesentlichen gleichförmig darin verteilter biologisch abbaubarer Keramik ausgewählt aus der Gruppe bestehend aus Calciumsulfat und Calciumphosphat.

2. Das Implantatmaterial nach Anspruch 1, welches im Wesentlichen nicht porös ist.

3. Das Implantatmaterial nach Anspruch 1, wobei das biologisch abbaubare Polymer ein Copolymer aus Polymilchsäure und Polyglycolsäure umfasst.

4. Das Implantatmaterial nach Anspruch 1, wobei die biologisch abbaubare Keramik sich zweimal so schnell zersetzt wie das biologisch abbaubare Polymer.

5. Das Implantatmaterial nach Anspruch 1, wobei die Partikelgröße der biologisch abbaubaren Keramik anfänglich zwischen 100 und 2000 µm ist.

6. Das Implantatmaterial nach Anspruch 2, welches eine mechanische Eigenschaft hat ähnlich der entsprechenden inhärenten mechanischen Eigenschaft des aufnehmenden Gewebes.

7. Das Implantatmaterial nach Anspruch 1, ebenfalls aufweisend biologisch abbaubare Fasern.

8. Das Implantatmaterial nach Anspruch 1, welches mit der Hand formbar ist zwischen Körpertemperatur und 55°C.

9. Verfahren zum Herstellen eines biologisch abbaubaren Implantatmaterials nach Anspruch 1, bei welchem man:
a) Partikel eines ausgewählten biologisch abbaubaren Polymers, das ein Molekulargewicht zwischen 25 000 und 1 000 000 Daltons hat, mit Partikeln einer ausgewählten biologisch abbaubaren Keramik ausgewählt aus der Gruppe bestehend aus Calciumsulfat und Calciumphosphat mischt; und
b) Hitze und Druck auf die Mischung anwendet um ein im Wesentlichen gleichförmig kohäsives Implantatmaterial herzustellen.

10. Verfahren nach Anspruch 9, wobei die Temperatur zwischen 75 und 100 °C ist.

## Revendications

1. Matériau pour implant biodégradable ayant un profil de dégradation biphasique choisi comprenant un polymère biodégradable ayant une masse moléculaire comprise entre 25 000 et 1 000 000 daltons et ayant une céramique biodégradable distribuée sensiblement de manière uniforme à l'intérieur choisie dans le groupe constitué par le sulfate de calcium et le phosphate de calcium.

2. Matériau pour implant selon la revendication 1, étant sensiblement non poreux.

3. Matériau pour implant selon la revendication 1, dans lequel ledit polymère biodégradable comprend un copolymère d'acide polylactique et d'acide polyglycolique.

4. Matériau pour implant selon la revendication 1, dans lequel ladite céramique biodégradable se dégrade deux fois plus vite que le polymère biodégradable.

5. Matériau pour implant selon la revendication 1, dans lequel ladite taille de particule de la céramique biodégradable est initialement comprise entre 100 et 2 000 µm.

6. Matériau pour implant selon la revendication 2, ayant une propriété mécanique similaire à la propriété mécanique intrinsèque correspondante du tissu receveur.

7. Matériau pour implant selon la revendication 1, comprenant également des libres biodégradables.

8. Matériau pour implant selon la revendication 1, étant façonnable à la main entre environ la température corporelle et 55 °C.

9. Procédé de fabrication d'un matériau pour implant biodégradable selon la revendication 1, comprenant les étapes consistant à :
a) mélanger les particules d'un polymère biodégradable choisi ayant une masse moléculaire comprise entre 25 000 et 1 000 000 daltons avec les particules d'une céramique biodégradable choisie, choisie dans le groupe constitué par le sulfate de calcium et le phosphate de calcium ; et
b) appliquer de la chaleur et une pression audit mélange pour produire un matériau pour implant cohésif sensiblement uniforme.

10. Procédé selon la revendication 9, dans lequel ladite température est comprise entre 75 et 100 °C.
